Europäisches Patentamt
European Patent Office
Office européen des brevets

(11) Publication number: 0 422 657 A1

# EUROPEAN PATENT APPLICATION

(21) Application number: 90119529.7

(22) Date of filing: 11.10.90

(51) Int. Cl.5: A61J 1/00

(30) Priority: 13.10.89 US 421249

(43) Date of publication of application: 17.04.91 Bulletin 91/16

(84) Designated Contracting States: AT BE CH DE DK ES FR GB GR IT LI LU NL SE

(71) Applicant: INTERNATIONAL MEDICATION SYSTEMS LTD
1886 Santa Anita Avenue
South El Monte California 91733(US)

(72) Inventor: Braddock Ogle II, George
5616 Bonita Ave
Alta Loma, CA 91701(US)

(74) Representative: Patentanwälte Grünecker, Kinkeldey, Stockmair & Partner
Maximilianstrasse 58
W-8000 München 22(DE)

## (54) Apparatus for storing, mixing, and using medication.

(57) Medication which is stored dry (62) or in a concentrated liquid form, and which must be mixed with a diluent (81) before use, is stored in a medication container (20) which has an opening for transferring medication to and from the container (20). A sealing member (26) over the container opening has an elongated passageway (35) extending through it to permit flow of material to and from the container (20). A removable closure (39) in the passageway (35) prevents flow of material until the medication is ready for use. A tapered connector (36) on the sealing member (26) surrounds the passageway (35), and is adapted to receive a mating tapered connector on another container into which material may be transferred from the container (20) after removing the closure (39) from the passageway (35).

A medical injector (70) includes a cylindrical vial (72) having an open end (74) and a closed end (76). A resilient plug (78) is press-fitted into the open end of the vial so a liquid diluent (81) can be stored in a sterile condition in the vial (72). An elongated barrel (88) includes a sealing member (90) at one end, and the sealing member (90) has a passageway (92) extending through it. An elongated cannula (94) is connected at one end to the passageway (92). The other end of the cannula (94) terminates in a scarf (98) in the vicinity of the plug (78). A threaded fitting (84) on the other end (86) of the barrel (88) receives mating threads (82) on one end of the plug (78) extending toward the barrel (88). The vial (72) and cannula (94) are first held in an assembled, but nonoperating, position when the threaded barrel (88) is coupled to the plug (78). Upon further interlocking of the plug (78) with the barrel (88), the scarf end (98) of the cannula (94) pierces the plug (78) so liquid (81) in the vial (72) can flow through the cannula (94) and out the passageway (92) in the sealing member (90). A tapered connector on the sealing member (90) and around the passageway (92) is adapted to receive a mating tapered connector (36) on the medication container (20) so that material can be transferred back and forth between the vial interior (72) and the container (20) by reciprocating the plug (78) in the vial (72).

EP 0 422 657 A1

Fig. 6

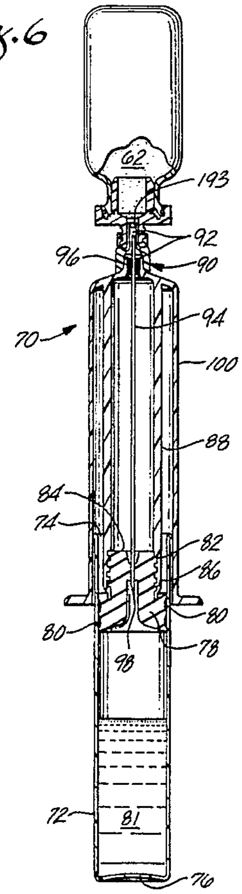

# APPARATUS FOR STORING, MIXING, AND USING MEDICATION

## BACKGROUND OF THE INVENTION

This invention relates to apparatus for storing, mixing, and using medication which is normally kept in a dry or concentrated state until just before use. Such medication which must be dissolved or dispersed in a liquid diluent or solvent before use, is more effective if stored in a dried or concentrated form, and not dissolved or diluted until just before being used.

After the concentrated medication is dissolved or dispersed in a liquid, e.g., sterile water or saline solution, that preparation must be transferred to a hypodermic syringe, or a similar device, fitted with a hypodermic needle or cannula (hollow needle) with a scarf (sharp end) for subsequent direct injection into a patient, or for addition to a Y-site or an intravenous (I.V.) solution bottle or bag, to permit infusion of the drug to the patient. The mixing of such medication with solvent or diluent presents many problems in proportioning and maintaining sterile conditions. U.S. Patents Nos. 3,376,866 and 3,542,023 to R. W. Ogle, 4,392,851 to A. M. Elias, and 4,516,967 to R. J. Kopfer are examples of prior art efforts to solve the problem of handling medication which must be stored in concentrated form until just before use. Those prior art devices require the use of exposed hypodermic needles or sharp spikes, which are dangerous because of possible unintended finger and hand punctures from accidental contact with the hypodermic needle or sharp spike, resulting in possible exposure to hazardous drugs or to dangerous viruses, such as hepatitis or AIDS (acquired immune deficiency syndrome) carried by the hypodermic needle or spike.

Moreover, the hypodermic needles of the prior art devices are used for both mixing and injection of the medication into a patient, a Y-site, or an I.V. bag. Accordingly, only relative small-diameter needles can be used, and that often makes mixing slow and difficult. The sharp spikes of the prior art devices have large internal diameters, which makes mixing faster and easier, but the large spike cannot be used on a patient, or repeatedly at a Y-site, or the like.

The prior art devices are also subject to accidental leakage or spillage of dangerous drugs onto the skin of personnel, or onto work surfaces.

## SUMMARY OF THE INVENTION

This invention provides improved apparatus for storing, mixing, and using medication which has to be diluted or dissolved just before use. The apparatus of this invention avoids the need for using an exposed needle or spike for mixing the medication, and when the mixed medication is to be used, it can safely and quickly be placed in a hypodermic syringe or the like, which can be connected to a conventional hypodermic needle, or to a protected cannula for safe injection into a Y-site or an I.V. bag. In either case, the hypodermic needle can be of relatively small diameter to minimize patient discomfort, or damage to the Y-site or I.V. bag.

Briefly, this invention provides a medication container which includes a hollow body having an opening for transferring medication to and from the body. A sealing member is disposed over the body opening for sealing the body interior from contamination. The sealing member has an elongated passageway extending through it to permit flow of material to and from the body interior. A removable closure in the passageway prevents flow of material to and from the body interior until closure is removed. A tapered connector on the sealing member and disposed around the passageway of the sealing member is adapted to receive a mating tapered connector on another container into which material may be transferred from the body interior after removal of the removable closure from the passageway.

Preferably, the container body is a bottle with a cylindrical opening or neck, and the opening through the neck is tapered slightly inwardly toward the body interior. The sealing member includes an annular skirt tapered to make a press fit in the bottle neck. An annular web on the sealing member fits over the outer end of the neck of the bottle. Threads are provided around the tapered connector for engaging mating threads on the other container. In the presently preferred embodiment, the tapered connector on the sealing member is a socket which tapers inwardly toward the container interior, and is surrounded by external or male threads to mate with threads on the other container.

The preferred form of the sealing member includes an annular lip around the outer periphery of the flange and extending toward the container interior to make a tight friction fit around an annular lip on the outer end of the bottle neck. Preferably, an annular groove facing the bottle interior is formed in the flange where the flange joins the annular skirt of the sealing member to provide improved flexibility for press-fitting the sealing member onto the bottle neck, which has an outwardly opening annular groove facing the interior surface of the sealing member lip to provide improved sealing pressure between the bottle neck lip and the sealing member lip.

In the preferred form, the removable closure is a removable diaphragm mounted across the passageway of the sealing means to prevent flow of material to and from the body interior until such time as the diaphragm is ruptured or removed.

The invention also includes a medication injector adapted to connect to the medication container so that a diluent or solvent in the medication injector can be mixed with medication in the medication container. The injector includes a cylindrical vial having an open end and a closed end. A resilient plug is press-fitted in the open end of the vial. An elongated barrel carries a sealing member at one end of the barrel, and the sealing member has a passageway extending through it. An elongated cannula is connected at one end to the passageway of the sealing member, and the other end of the cannula terminates in a scarf in the vicinity of the plug. The other end of the elongated barrel carries interlocking means adapted to fit with cooperating interlocking means on the plug so that, upon interlocking the plug with the barrel, the vial and cannula are first held in an assembled, but nonoperating, position and, upon further interlocking of the plug with the barrel, the scarf end of the cannula pierces the plug to provide communication between the vial interior and the passageway through the sealing member. A tapered connector on the sealing member and around the passageway projects away from the vial and beyond the end of the cannula connected to the passageway of the sealing means. The tapered connector is adapted to receive the tapered connector on the medication container so material can be transferred back and forth between the vial interior and the container by reciprocating the plug in the vial. In the preferred form, the medication injector includes threads around the tapered connector. Preferably, the tapered connector is a nozzle which tapers inwardly in a direction away from the cylindrical vial, and which is surrounded by internal threads spaced from the tapered nozzle of the connector. Preferably, the tapered connector is also adapted to be connected to a conventional hypodermic needle or to a cannula protected by a sheath to prevent accidental sticking.

In the preferred form, the protected cannula includes an elongated sheath having a bore extending through it. The cannula with a scarf end is mounted and sealed in the bore of the sheath so the scarf end of the cannula is located within an adjacent one end of the sheath. The end of the sheath adjacent the scarf end of the cannula has at least one cutout portion which can receive the edge of a flexible bag, or flexible tubing connected to a Y-site for intravenous injection. The sheath includes a socket at the end of the cannula remote from the scarf. The socket has an opening through it so the socket interior is connected to the passage through the cannula. The socket opening tapers outwardly away from the cannula for receiving a tapered nozzle on a syringe or the like. In the preferred form, the sheath, which is preferably cylindrical, includes two diametrically opposed cutouts adjacent the scarf end of the cannula, and longitudinal grooves are provided on the outside of the sheath to provide improved gripping for manipulating the device. Preferably, the socket carries laterally extending ears in the shape of interrupted male threads for engaging an internally threaded skirt on a syringe or the like. The skirt surrounds a tapered nozzle projecting from the syringe, and is spaced from the nozzle. Preferably, the sheath is disposed around and spaced from the ears and outwardly tapering end of the socket to form an annular space for receiving the skirt on the syringe. The outer surface of the skirt makes a friction slip-fit against the inside of the socket, and the outer surface of the skirt is irregular to minimize the possibility of accidental disengagement of the socket from the syringe.

These and other aspects of the invention will be apparent from the following detailed description, taken in conjuction with the accompanying drawings.

## DESCRIPTION OF THE DRAWINGS

FIG. 1 is an elevation, partly in cross section, of the presently preferred medication container of this invention with a cover mounted on the container;

FIG. 2 is an elevation of the container cover removed from the container;

FIG. 3 is a fragmentary elevation, partly in cross section, of the medication container shown in FIG. 1, but with the cover removed;

FIG. 4 is an elevation, partly in cross section, of the presently preferred medication injector with a cover mounted over one end of the injector;

FIG. 5 is an elevation of the cover shown in FIG. 4, but removed from the injector;

FIG. 6 is an elevation, partly in cross section, showing the medication container connected to the medication injector;

FIG. 7 is an elevation of the medication injector connector to a protected cannula assembly;

FIG. 8 is a view taken on line 8-8 of FIG. 7, but with the injector separated from the protected cannula assembly;

FIG. 9 is a view taken on line 9-9 of FIG. 8;

FIG. 10 is a view taken on line 10-10 of FIG. 9;

FIG. 11 is a view taken on line 11-11 of FIG. 8;

FIG. 12 is an elevation of the medication injector connected to the protected cannula, which is connected to a Y-site; and

FIG. 13 is an elevation of the medication injector connected to the protected cannula, which is in the process of being connected to an I.V. bag.

## DETAILED DESCRIPTION

Referring to FIGS. 1-3, a medication container 20 includes a bottle 21 which has a cylindrical body 22 and a cylindrical neck 23 of smaller diameter than the bottle body. The interior annular surface 24 of the bottle neck is conical and tapers inwardly slightly in the direction toward the bottle interior.

A sealing member 26 includes an annular skirt 27 which tapers inwardly slightly to make a snug friction fit in the bottle neck. An annular flange 28, in the shape of an annula disc formed intergrally with the outer end of the skirt, fits over the outer end of the bottle neck, and carries an annular flange lip, which extends toward the bottle and makes a snug friction fit around an outwardly extending bottle lip 30 on the outer end of the bottle neck. An outwardly extending annular rib 31 formed integrally with the outer surface of the flange adjacent the free edge of the flange provides additional gripping strength of the flange around the bottle neck lip. An annular groove 32 in the flange surface facing the bottle neck lip, and in the vicinity of where the flange joins the annular skirt 27, provides improved flexibility for the web and flange to permit a tight fit of the flange lip around the bottle neck lip. An outwardly facing annular groove 33 in the outer surface of the skirt near where it joins the flange also facilitates flexibility of the flange and web for making a tight clamping fit on the bottle neck lip. An outwardly facing annular groove 34 in the bottle neck lip provides increased pressure of the interior surface of the flange against the exterior surface of the bottle neck lip for improved sealing.

The sealing member includes an elongated passageway 35 extending through it to permit flow of material to and from the interior of the bottle. An elongated tubular connector 36 formed integrally with the upper surface of the sealing member extends away from the bottle, and includes a straight bore 37, which opens down into a large bore 38 extending through the skirt. The upper end of bore 37 is closed by an imperforate diaphragm 39 formed integrally with the interior surface of the connector 36.

The outer end of the tubular connector includes a socket 40, which tapers inwardly toward the bottle interior, and terminates at its inner end at the diaphragm, which is adapted to be removed or perforated, as described below.

Male threads 42 on the external surface of the connector 36 extend from where the connector joins the flange to a point spaced from the outer end of the connector by a distance equal to about one-half the diameter of the tapered socket 40 to leave an outwardly extending unthreaded portion 43 to facilitate rupturing or removal of the diaphragm, as described below. The diaphragm, which serves as a removable closure in the passageway 35 formed by collinear bores 37, 38, and 40, prevents flow of material to and from the body interior until the diaphragm is ruptured or removed. Alternatively, the diaphragm can be omitted, and the passageway 35 may be closed by an elongated stinger (not shown) on the interior surface of a cover 44, which makes a tight sealing fit over the sealing member mounted on the neck of the bottle. As shown in FIGS. 1 and 2, the cover 44 includes a domed outer end 48 formed integrally with a cylindrical section 50, which includes longitudinally extending grooves 52 to facilitate gripping of the cover. The end of the cylindrical section 50 nearer the bottle includes an annular and outwardly extending cover web 53 which rests on the outer surface of the sealing member flange 28. An annular flange 54 is formed integrally at one edge with the outer periphery of the cover web 53, and makes a tight fit around the outer surface of the sealing member lip 29. The annular rib 31 on the sealing member flange ensures firm gripping of the cover to the sealing member. The inside diameter of the cylindrical section 50 of the cover is larger than the exterior diameter of the external threads on the tapered connector of the sealing member. An inwardly extending short, cylindrical stinger 60 is formed integrally at its outer end with the interior of the dome 48 of the cover, and may be replaced by a longer stinger (not shown) which tapers inwardly in the direction of the bottle to make a snug fit in the tapered socket 40, if the diaphragm is omitted. In that case, the longer stinger would serve as a removable closure in the passageway 34, and prevent medication in the bottle from inadvertently flowing into the space between the cover and the sealing member. The same function is provided by the removable or rupturable diaphragm. Either arrangement avoids loss of medication when the cover is removed. Such is loss is to be avoided, not only from the standpoint of economy, but also because the medication may be toxic and dangerous if improperly ingested. The short stinger 60, shown in FIG. 1, is provided to serve as a closure member when the connector 36 of FIG. 3 may be replaced by one of the type shown in FIG. 8, as explained below.

The medication container components, i.e., the bottle, sealing member, and cap, can all be made of any suitable plastic approved for medication to be stored in the container. Alternatively, the bottle can be made of glass, if liquid is to be stored in it, but the sealing member should be made of an elastomeric rubberlike material, or at least an annular rubberlike member (not shown) should be disposed between the bottle and the plastic parts of the sealing member to provide the seal required for liquid.

Referring to FIGS. 4, 5, and 6, a medication injector 70 includes an elongated cylindrical vial 72 having an open end 74 and a closed end 76. A resilient plug 78 is press-fitted in the open end of the vial. A pair of outwardly facing and longitudinally spaced annular grooves 80 in the plug ensure a good sealing fit between the plug and the interior surface of the vial, and facilitate relative reciprocal movement between the vial and the plug, in the manner described below. Ordinarily, the vial contains a solvent or diluent 81, such as sterile water or sterile saline solution for mixing with the medication 62 in the medication container. The outer end of the resilient plug is of reduced diameter and carries external threads 82 adapted to mate with female threads 84 in the inner end 86 of an elongated cylindrical barrel 88. The outer end of the barrel carries an outwardly extending and cylindrical sealing member 90 having a longitudinal passageway 92 extending through it. An elongated cannula 94 is sealed by epoxy resin 96, or the like, at one end in the sealing member passageway 92. The other end of the elongated cannula terminates in a scarf 98, which initially is embedded in the upper portion of the resilient plug, as shown in FIG. 4. Also, as shown in FIG. 4, the threads on the outer end of the resilient plug are only partially engaged with the threads on the inner end of the barrel so that the cannula does not completely pierce the plug. A cylindrical guide 100 is disposed around and spaced from the barrel exterior, and is formed at its outer end integrally with the outer end of the barrel. The inner end of the guide carries an outwardly extending flange 102, which serves has a handle, as described below. The open end of the vial makes a telescoping and sliding fit between the guide and the barrel. As shown in FIG. 4, the sealing member on the outer end of the barrel is covered by an elongated and domed cover 104 which makes a snug friction fit around the exterior of the sealing member to keep it clean and sterile until it is ready for use, as shown in FIG. 6. FIG. 5 is an elevation of the injector cover, which has longitudinally extending ribs 105 in its intermediate portion and external annular grooves 106 perpendicular to the longitudinal ribs 105 and located between the outer ends of the ribs and the domed end of the cover.

To use the medication container and medication injector to mix the dry powder 62 in the container with the liquid in the injector, the respective covers are removed from the container and the injector. The threaded connector on the medication connector is threaded into the injector sealing member 90 (the detailed construction of which is explained in more detail below with respect to FIG. 8). However, since the external threads on the sealing member of the medication container terminate short of the outer end of the medication container connector, the container connector has to be inserted a substantial distance into the injector sealing member before the container and injector sealing members are properly engaged. This forces the diaphragm 38 in the medication container sealing member to be ruptured or removed before the two sealing members are fully engaged. This avoids the possibility of the two sealing members becoming partially engaged before the diaphragm is ruptured and misleading the user to believe that proper connection has been made when, in fact, it has not.

The use of the assembly shown in FIG. 6 to mix the powdered medication and the liquid is described below.

When mixing is completed, the mixture is stored in that portion of the vial between the stopper and the closed end of the vial. The medication container is unscrewed from the sealing member of the injector, which is then threaded into one end of a protected cannula assembly 169, as shown in FIG. 7.

FIGS. 8-11 show in detail how the protected cannula assembly 169 may be securely attached to the sealing member 90 on the discharge end of the medication injector 70. FIG. 8 is taken on line 8-8 for FIG. 7, but the cannula assembly 169 and the sealing member 90 on the injector are shown disengaged and slightly separated. Referring to FIGS. 8-11, a cannula 170 is press-fitted and sealed with adhesive (not shown) in a longitudinal bore 171 through an annular boss 174, which is press-fitted and sealed with an adhesive (not shown) in a recess 176 in an annular insert 178 press-fitted and sealed with an adhesive (not shown) in a longitudinally extending central stepped bore 180 through a cylindrical protective sheath 182, which has diametrically opposed cutouts 184 at the end of the sheath surrounding the scarf end 186 of the cannula. The slots 184 are sufficiently narrow that it is unlikely a nurse, doctor, or technician could accidentally touch the cannula scarf, and yet each slot 184 is sufficiently wide and deep to accommodate and receive a laterally extending tubing of a conventional Y-site connection, as shown in FIG. 1 of my U.S. Patent 4,834,716. The cannula may be of any appropriate size, but since it is not used for mixing, it can be relatively small, say, about 18-gauge, so it can be used to puncture a Y-site or I.V. bag connection repeatedly without destroying the self-sealing property of the connection.

The end 188 of the cannula opposite from the scarf is squared off and bears against an inwardly extending annular shoulder 189 of the insert 178, which has a coaxial bore 190 which connects the bore (not shown) through the cannula with an enlarged and longitudinally extending socket, or recess, 192 in the end of the insert remote from the scarf. Recess 192 is of conical shape and tapers outwardly away from the cannula to an angle to match that of a tapered discharge nozzle 193 at the outer end of sealing member 90 of the injector 70. The seating of the squared- off end of the cannula against the shoulder 189 accurately locates the cannula relative to the insert, and facilitates precision assembly of the protection device.

The exterior of insert 178 has an annular shoulder 194 which bears against a matching annular shoulder 195 in the bore 180 extending through the sheath. Thus, when the insert shoulder 194 seats on shoulder 195 of the sheath, the insert is properly positioned within the sheath to locate the scarf of the cannula and the ears of the insert in their required respective positions shown in FIG. 8 to provide precise alignment of the components which make up the device.

The end of insert 178 remote from the scarf of the cannula carries a pair of diametrically opposed and laterally extending ears 196. Each ear 196 is shaped as shown in FIG. 10 to form part of an interrupted male thread 198, which engages internal female threads 200 formed on the inside surface of an annular skirt 202 formed on the sealing member 90 to extend around, and be spaced from, the discharge nozzle 193, which has a coaxial stepped bore 204 to permit the discharge of liquid from the injector. The discharge nozzle 193 projects outwardly beyond the annular skirt 202 to facilitate inserting the nozzle into the tapered recess (socket) 192 of the insert 78. A plurality of longitudinally extending grooves 206 (FIG. 11) on the outer surface of the skirt 202 provide longitudinally extending ridges 208 spaced at intervals of about 6$^{\circ}$ around the circumference of the skirt.

When the injector nozzle 193 is inserted into the tapered recess 192 of insert 178, the interrupted male threads 198 on ears 196 engage the internal female threads 200 of the skirt 202, as in a conventional Luer lock of the type shown in U.S. Patent No. 4,737, 144 to Choksi. Rotation of the sheath and injector relative to each other causes the ears to follow the threads inwardly and draw the tapered nozzle 193 into a snug and sealed fit in the tapered recess 192. At the same time, the exterior surface of the skirt 202 makes a sliding friction fit against the inside surface 210 of an annular ring 212 formed integrally on the end of the sheath remote from the scarf end of the cannula and terminating flush with the outer end of insert 178. The annula ring 212 is spaced from the exterior of the insert around socket 192 to provide an annular space 213 which receives the skirt 202 as the injector is coupled to the cannula protective assembly 169. The annular ring 212 also provides backup for the annular skirt 202 and ensures snug, locking engagement of the ears 196 and the internal threads 200. In addition, the longitudinally extending ridges 208 on the exterior surface of the skirt 202 helps prevent inadvertent backing out or disengagement of the ears 196 from the internal threads. Thus, the protected cannula is easily and firmly secured to the discharge end of the injector, which was previously loaded with medication, as described below with respect to FIG. 6.

## MIXING OPERATION

The medication container and injector are threaded together as shown in FIG. 6. The external male threads 42 on the connector 36 of the container sealing member mate with the internal female threads 200 of the injector sealing member, and draw the tapered nozzle 193 firmly into the tapered socket 40. However, before the male and female threads can engage, the nozzle must be inserted into socket 40 for a distance sufficient to rupture diaphragm 38. Thereafter, a slight additional insertion of the nozzle 193 into the tapered socket 40 causes the internal threads 200 to engage the external threads 42. rotation of the container relative to the injector causes the threads to mate up tightly, and lock the nozzle in the socket. However, this cannot occur until after the diaphragm is ruptured, thus establishing communication between the interior of the container and the interior of the cannula. The vial is then rotated about its longitudinal axis so that the external threads on the rubber plug carry the plug into the position shown in FIG. 6, i.e., with the scarf end of the cannula projecting through the rubber plug and into the vial between the rubber plug and the closed end of the vial. The friction between the plug and the vial is sufficient to permit the threading operation just described, and cause the scarf end of the cannula to pierce the rubber plug. However, the friction between the plug and the vial is not so great that the vial cannot be rotated relative to the plug. Once the plug threads are firmly seated in the barrel threads, rotation of the vial relative to the rubber plug may be necessary to break any seizure of the plug to the wall of the vial. Thereafter, the vial can be reciprocated back and forth on the plug to cause the liquid in the vial to enter the medication container and mix with the powdered medication there. Of course, the medication in the medication chamber could be a concentrated liquid, rather than powder, but normally that medication is a powder. As shown in FIG. 6, the vial is not more than about two-thirds full of liquid, the remainder of the space between the liquid and the inner end of the rubber plug is filled with sterile air, which is displaced into the container as the vial is moved up into the annular space between the guide and the barrel. Continued movement of the vial in that direction until the bottom of the vial contacts the inner end of the rubber plug displaces virtually all of the liquid from the vial into the medication container. The vial can be retracted to draw the mixture from the container, this operation being facilitated by holding the assembly in the position shown in FIG. 6 so that the container is above the vial. It may be necessary to reciprocate the vial a few times to cause the liquid to surge back and forth between the vial and the medication container interior until the medication is thoroughly dispersed or dissolved in the liquid. Alternatively, the entire assembly can be shaken without any danger of inadvertent separation of the medication container from the injector because those two elements are locked together by the threaded connection at their respective connectors.

After the medication in the medication container is properly dispersed or dissolved, the mixture is withdrawn from the container into the vial by retracting the vial to about the position shown in FIG. 6. Thereafter, the medication container is unscrewed from the connector on the sealing member of the injector. Once the medication container is removed from the injector, the injector is then connected to the protected cannula assembly, as shown in FIG. 7 and described above with respect to FIGS. 8-11. The scarf end of the cannula can then be connected to a Y-site 215, with one of either of the two longitudinal slots 184 of the protective sheath straddling one branch of the Y, as shown in FIG. 12. Alternatively, the protected cannula and injector system can be connected to an I.V. bag 271, as shown in FIG. 13. In the view shown in FIG. 13, the protected cannula is not quite fully engaged with the I.V. bag. To complete the engagement, the protected cannula assembly is rotated 90° about its longitudinal axis so the longitudinal slots 184 of the protected cannula assembly will straddle the edge of the bag as the cannula is fully inserted into the I.V. bag.

Another use of the invention is shown in FIG. 14, in which the mixed medication is injected into the medication container 20, which is then connected to a conventional hypodermic syringe 220 having a plunger 221, and a tapered nozzle 222, which fits into the tapered socket 40 of the medication container connector. If desired, and as an example of yet another use of the invention, the syringe can first be partially filled with liquid (say, sterile water or saline solution), and then connected as shown in FIG. 14 to the container 20 which carries the dry medication in powder form. The syringe plunger 221 is then reciprocated to cause the liquid to surge back and froth between the container and the syringe until the medication is properly dissolved or dispersed in the liquid. The proper amount of mixed medication is then drawn into the syringe, which can be removed from the medication container 20, and connected to an appropriate conventional hypodermic needle, or other connection for use of the medication.

FIG. 15 shows yet another embodiment in which a conventional hypodermic syringe 224 includes a nozzle 226 surrounded by internal threads 228 which engage the external threads 42 on the medication container connector. In this case, the construction of the nozzle 226 and the surrounding internal threads 228 are identical with that shown in FIG. 8 so that the tapered nozzle 226 can be securely locked to the medication container during either transfer of premixed medication from the container to the syringe, or during mixing of powdered medication with liquid in the syringe, as described above with respect to FIG. 14. In either event, the syringe is filled by withdrawing the plunger 221, disconnected from the medication container, and connected to a conventional hypodermic needle, or an injection point at a Y-site or I.V. bag.

From the foregoing description, it will be clear that this invention permits dangerous medication to be mixed quickly and safely, and without the use of exposed hypodermic needles or sharp spikes. Moreover, once the mixing has been effected, the mixture can be safely transferred to a Y-site connection, an I.V. bag, or a conventional hypodermic syringe. An important advantage of the present invention is that the mixing takes place through a relatively large bore cannula, which has not been possible with the prior art devices such as those shown in U.S. patents Nos. 3,376,866 or 3,542,023 to Ogle, where the cannula through which the mixed solution passed was also used either for injecting a patient, a Y-site, or an I.V. bag connection. The maximum size hypodermic needle which can be used on a patient, or for repeated puncturing of a Y-site or the like, is about gauge 18 (0.0405" i.d.). Since the cannula in the medication injector of the present invention is not used on a patient, or for injection to a Y-site or an I.V. bag, the present invention can use a much larger cannula, say, 14-gauge, which has an i.d. of about 0.70", or almost twice the size of the maximum size hypodermic needle acceptable for human use or repeated puncture of a Y-site or the like. The 18-gauge cannula used in the protected cannula assembly of this invention can be used to pierce a Y-site connection or the like repeatedly without damaging that site.

## Claims

1. A medication container comprising:
a hollow body having an opening for transferring medication to and from the body;
a sealing member disposed over the body opening for sealing the body interior from contamination, the sealing member having an elongated passageway extending through it to permit flow of material to and from the body interior;
a removable closure in the passageway for preventing flow of material to and from the body interior; and
a tapered connector on the sealing member, the tapered connector being disposed around the passageway through the sealing member and adapted to receive a mating tapered connector on another container into which material may be transferred from the body interior after removal of the removable closure from the passageway.

2. A medication container according to claim 1 in which the hollow body is a bottle having an elongated cylindrical neck, and the sealing member includes an elongated annular skirt which fits into the neck of the bottle, the sealing member including a flange formed integrally with the skirt and extending over the bottle opening.

3 A medication container according to claim 1 or 2 which includes threads disposed around the tapered connector for receiving mating threads on another container.

4. A medication container according to claim 1 or 2 in which the tapered connector includes a tapered socket which tapers outwardly away from the body, and which includes external threads around the socket.

5. A medication container according to claim 2 which includes an annular groove in the flange in the vicinity of where the flange adjoins the skirt.

6. A medication container according to claim 5 in which the annular groove opens toward the bottle.

7. A medication container according to claim 2 in which the interior surface of the bottle neck tapers inwardly toward the interior of the bottle, and the annular skirt on the sealing member has a matching taper.

8. A medication container according to claim 2 which includes an outwardly opening annular groove around the bottle neck, and the flange includes an annular lip which makes a friction fit around the bottle neck and over the annular groove in the bottle neck.

9. A medication container according to claim 1 or 2 in which the removable closure is a diaphragm in the passageway through the sealing member.

10. A medication container according to claim 1 or 2 which includes a removable cover mounted over the sealing member.

11. A medication container according to claim 10 which includes detent means for securing the cover to the sealing member.

12. A medication injector comprising:
a cylindrical vial having an open end and a closed end;
a resilient plug press-fitted in the open end of the vial;
an elongated barrel;
a sealing member at one end of the barrel, the sealing member having a passageway extending through it;
an elongated cannula connected at one end to the passageway of the sealing member, the other end of the cannula terminating in a scarf in the vicinity of the plug;
interlocking means on the other end of the barrel;
cooperating interlocking means on the plug, whereby upon interlocking of the plug with the barrel, the vial and cannula are first held in an assembled but nonoperating position and, upon further interlocking of the plug with the barrel, the scarf end of the cannula pierces the plug to provide communication between the vial interior and the passageway through the sealing member; and
a tapered connector on the sealing member and around the passageway, the tapered connector of the sealing means projecting away from the vial and beyond the end of the cannula connected to the passageway of the sealing means, and the tapered connector being adapted to receive a mating tapered connector on a container into which material can be transferred back and forth between the vial interior and the container by reciprocating the plug in the vial.

13. A medication container according to claim 12 which includes threads disposed around the tapered connector.

14. A medication container according to claim 12 in which the tapered connector includes a nozzle tapered inwardly in a direction away from the vial, and having internal threads disposed around and spaced from the tapered nozzle.

15. A medication mixing system comprising:
a hollow body having an opening for transferring medication to and from the body;
a sealing member disposed over the body opening for sealing the body interior from contamination, the sealing member having an elongated passageway extending through it to permit flow of material to and from the body interior;

a tapered connector on the sealing member, the tapered connector being disposed around the passageway through the sealing member and adapted to receive a mating tapered connector on another container into which material may be transferred from the body interior after removal of the removable closure from the passageway;
a cylindrical vial having an open end and a closed end;
a resilient plug press-fitted in the open end of the vial;
an elongated barrel;
a sealing member at one end of the barrel, the sealing member having a passageway extending through it;
an elongated cannula connected at one end to the passageway of the sealing member, the other end of the cannula terminating in a scarf in the vicinity of the plug;
interlocking means on the other end of the barrel;
cooperating interlocking means on the plug, whereby upon interlocking of the plug with the barrel, the vial and cannula are first held in an assembled but nonoperating position and, upon further interlocking of the plug with the barrel, the scarf end of the cannula pierces the plug to provide communication between the vial interior and the passageway through the sealing member; and
a tapered connector on the sealing member and around the passageway, the tapered connector of the sealing means projecting away from the vial and beyond the end of the cannula connected to the passageway of the sealing means, and the tapered connector being adapted to receive the mating tapered connector on the medication container into which material can be transferred back and forth between the vial interior and the container by reciprocating the plug in the vial.

16. A medication mixing system according to claim 15 which includes a removable diaphragm in the passageway for preventing flow of material to and from the body interior, and the two tapered connectors being constructed and arranged so that when they are mated together, the diaphragm is ruptured to permit material to flow between the container and the vial.

17. A medication mixing system according to claim 16 which includes respective mutually engageable threaded portions for the two tapered connectors, the threaded portions and the tapered connections being constructed and arranged so that the diaphragm is ruptured before the threaded portions can be engaged.

Fig. 1

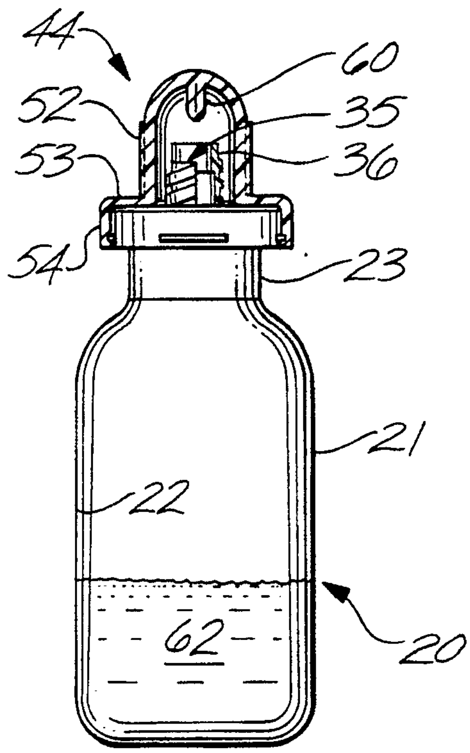


Fig. 2

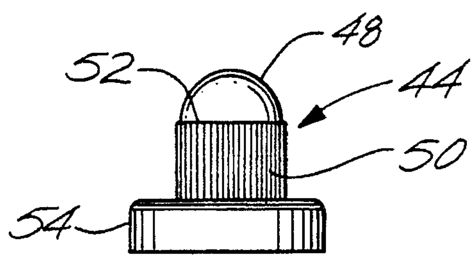

Fig. 3

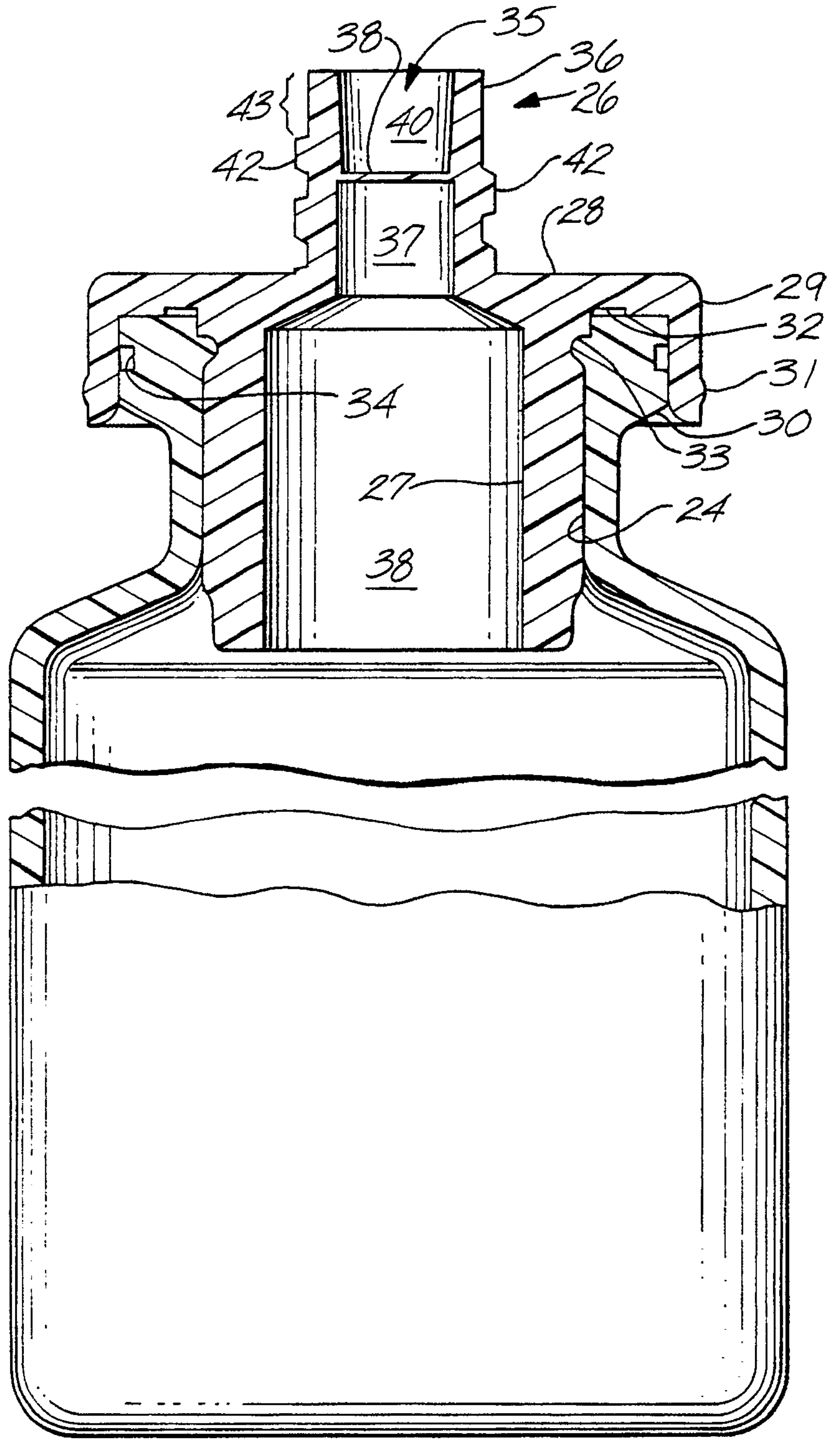

Fig. 4

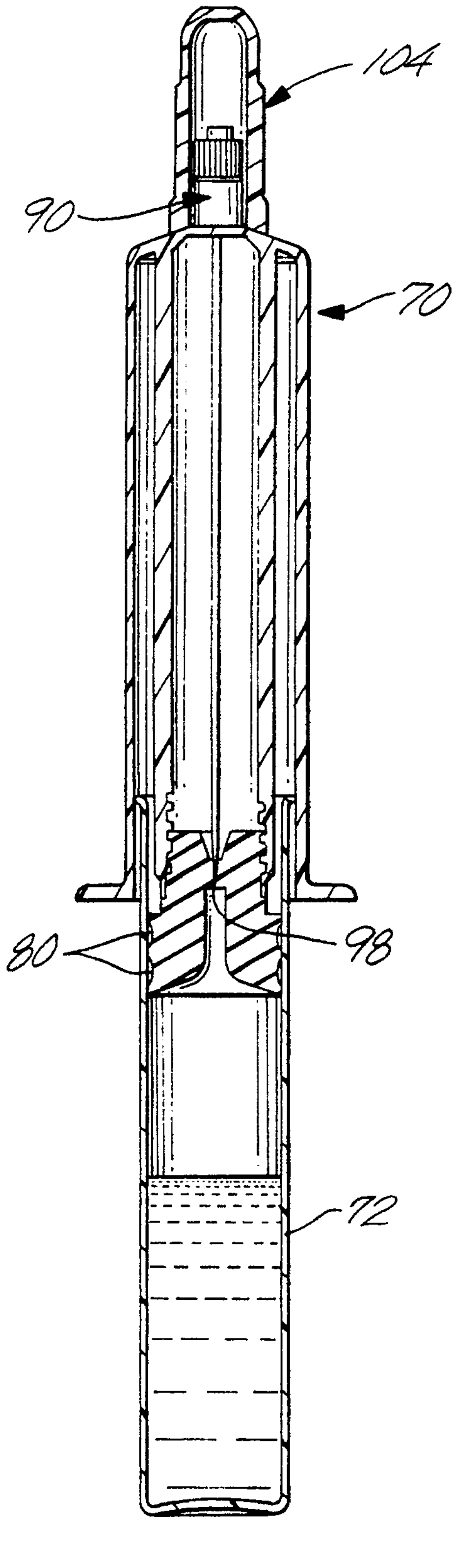


Fig. 5

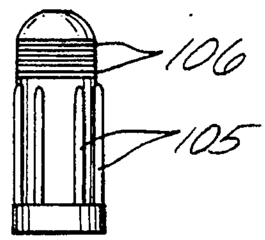

Fig. 6

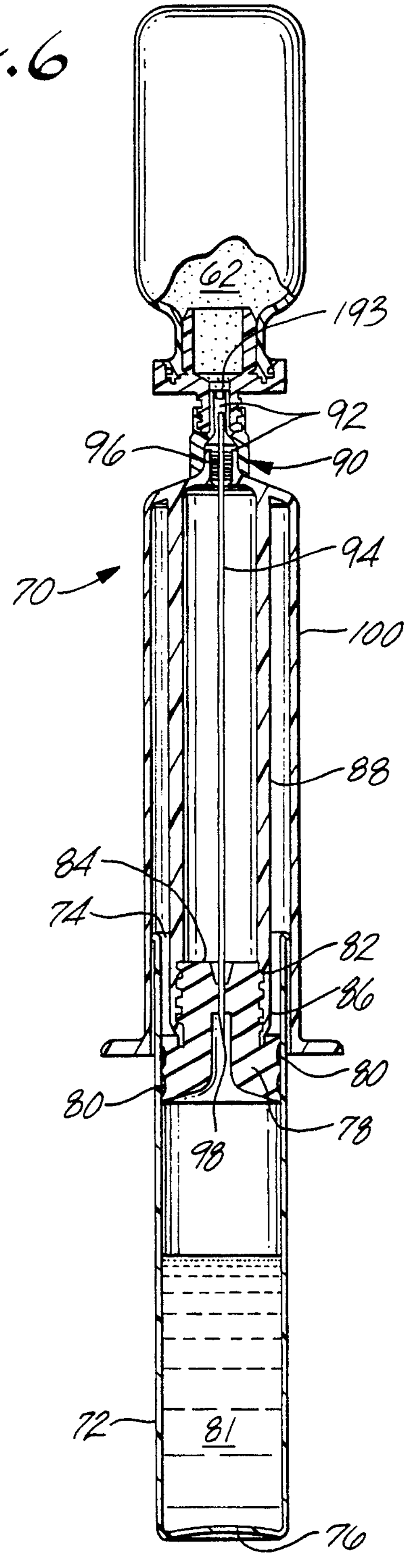

Fig.7

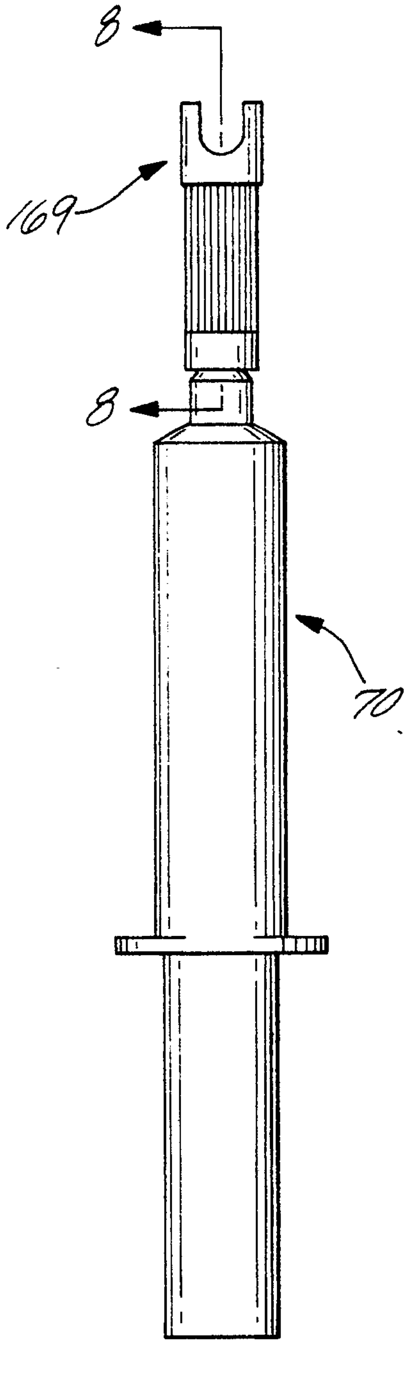


Fig.12

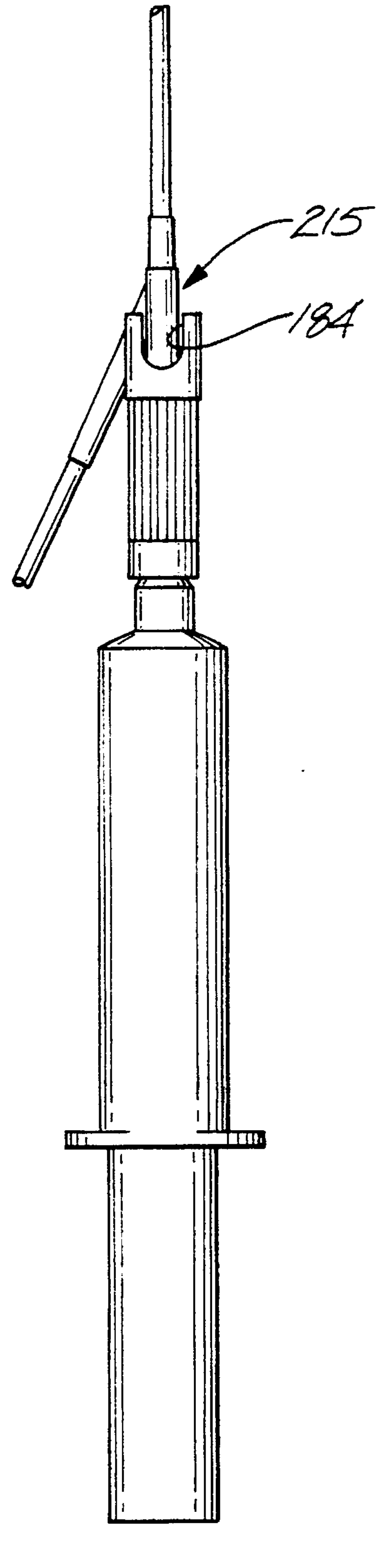

Fig. 9

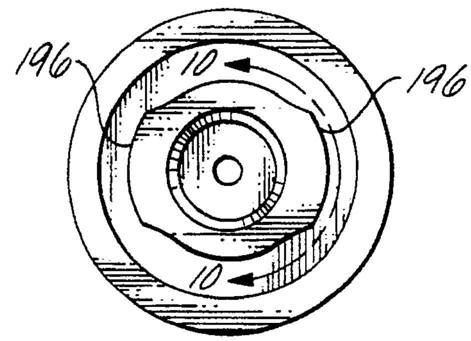


Fig. 10

Fig. 8

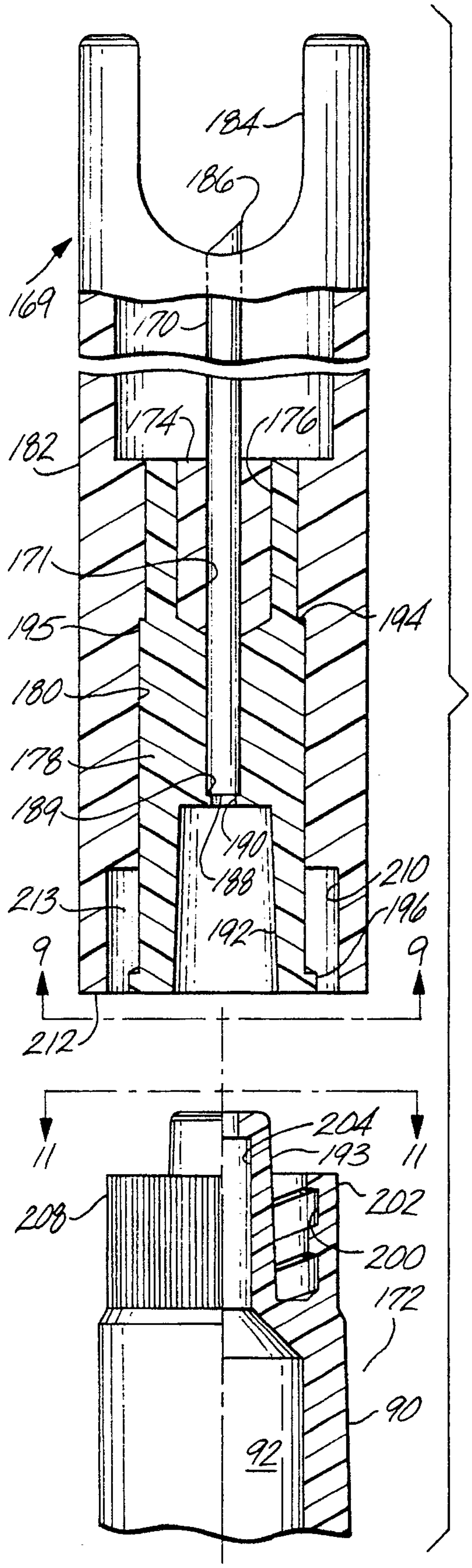


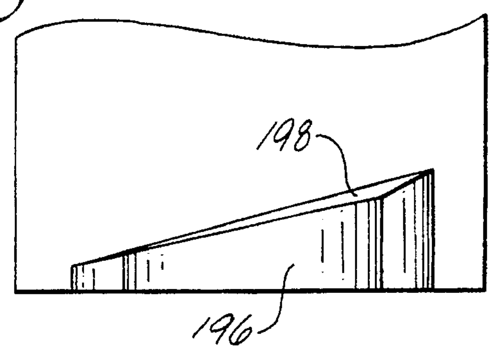


Fig. 11

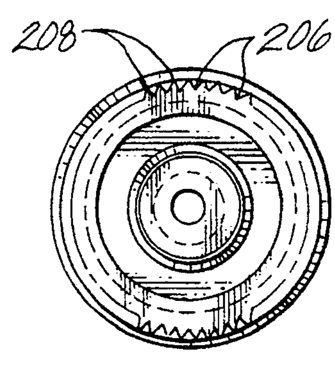

Fig. 13

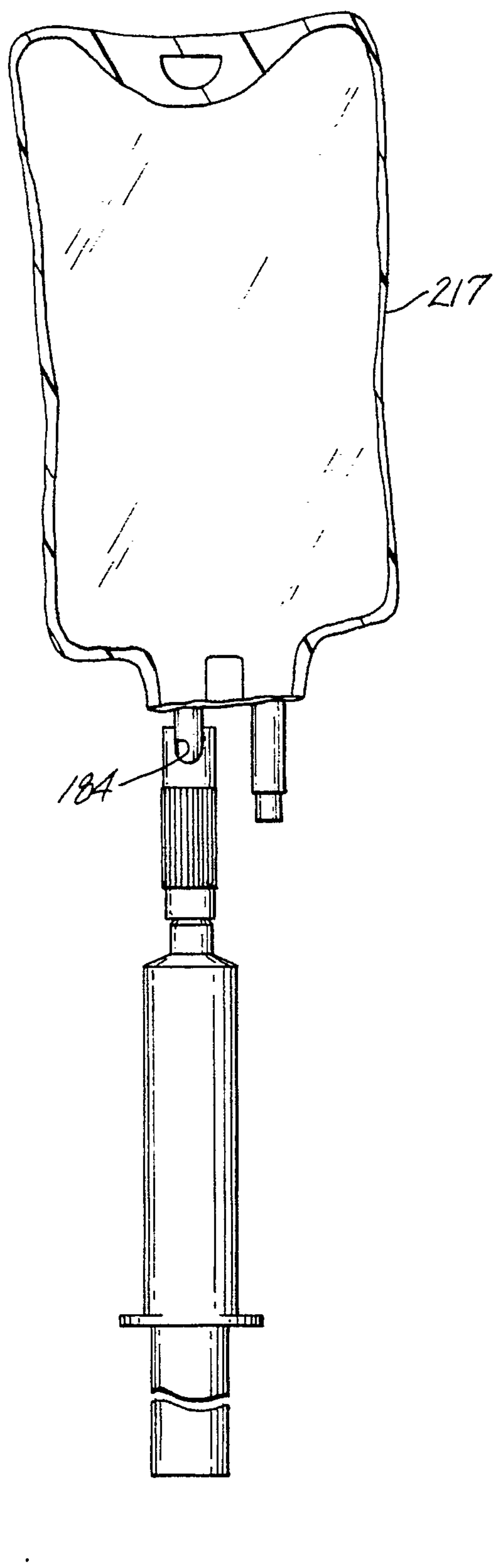

Fig. 14

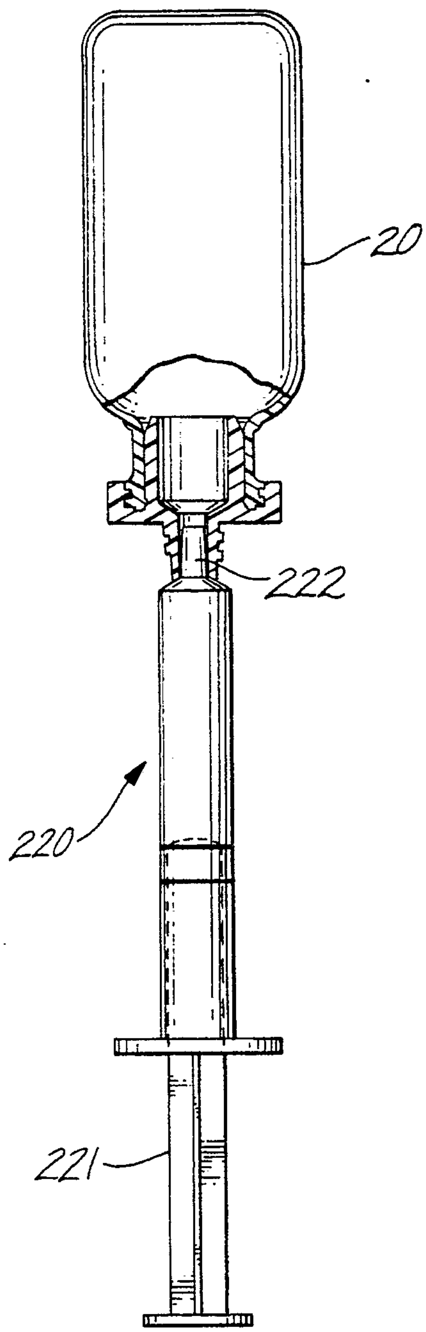


Fig. 15

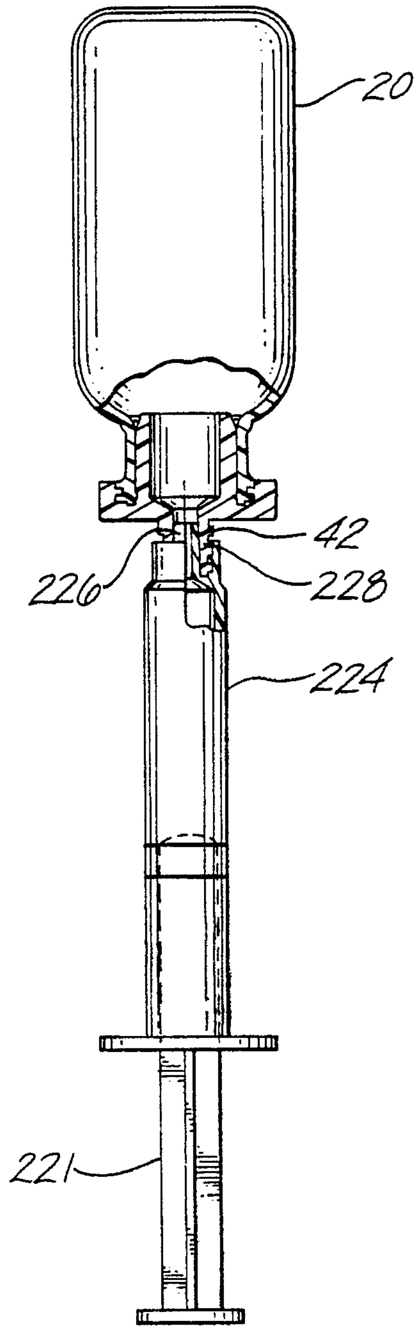

European
Patent Office

# EUROPEAN SEARCH REPORT

Application Number

**EP 90 11 9529**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FR-A-2 427 960 (DEHAIS)<br>* Claim 1; figures 1-3 *<br>- - - | 1-3,7-10 | A 61 J 1/00 |
| Y | | 4-6,15,16 | |
| Y | US-A-4 172 457 (CHOKSI et al.)<br>* Column 4, lines 30-36; figures 1-5 *<br>- - - | 4 | |
| Y,D | US-A-4 392 851 (ELIAS)<br>* Figures 4,5; column 2, line 67 - column 3, line 2 *<br>- - - | 5,6 | |
| Y | NL-A-7 214 588 (IMS LTD)<br>* Figures 2,3 *<br>- - - | 12-16 | |
| Y | US-A-3 659 602 (CLOYD)<br>* Figures 1,2 *<br>- - - | 12-14 | |
| A | US-A-4 153 057 (KÖBEL)<br>* Figures 1-7 *<br>- - - - - | 1,12,15 | |

TECHNICAL FIELDS SEARCHED (Int. Cl.5)

A 61 J
A 61 M

The present search report has been drawn up for all claims

| Place of search | Date of completion of search | Examiner |
|---|---|---|
| The Hague | 10 December 90 | GODOT T.G.L. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document